# EUROPEAN PATENT APPLICATION

(11) **EP 4 252 691 A1**
(43) Date of publication of application: **04.10.2023**
(21) Application number: 23164435.2
(22) Date of filing: 27.03.2023
(51) Int. Cl.: A61B 18/12, A61B 90/00

(54) **ELECTROSURGICAL GENERATOR WITH A SINGLE USE ACTIVATION COUNTER, ELECTROSURGICAL GENERATOR SYSTEM AND METHOD FOR OPERATING AN ELECTROSURGICAL GENERATOR**

(30) Priority: 28.03.2022 US 202263324344 P
(71) Applicant: Olympus Winter & Ibe GmbH, 22045 Hamburg (DE)
(72) Inventor: Janich, Fabian, 14469 Potsdam (DE); Kwik, Anne, 14165 Berlin (DE); Krüger, Jens, 15738 Zeuthen (DE); Horn, Martin, 22175 Hamburg (DE)
(74) Representative: Eisenführ Speiser

(57) **Abstract**

The invention relates to an electrosurgical generator for operating an electrosurgical instrument, characterized in that the electrosurgical generator comprises a data storage device coupled to the control device for storing a warning time and a switch-off time, the warning time and the switch-off time each being assigned to a detected instrument in order to assign an instrument-specific warning time and switch-off time to each detected instrument, a time tracking device for determining an instrument-specific activation duration, wherein the activation duration is a duration of time detected from a first connection or a first activation of an electrosurgical instrument connected to the instrument terminal to an actual time value, and wherein an activation duration is detected for each detected instrument, and the activation duration is recorded continuously and independently of an operating state of the electrosurgical instrument, and the control device triggers the warning indication by triggering the signaling device when the determined activation duration exceeds the instrument-specific warning time of the connected instrument, and/or the control device triggers the taking or keeping the connected instrument out of operation by controlling the deactivation device when the determined activation duration exceeds the instrument-specific switch-off time. The invention also relates to an electrosurgical generator system and a method for operation an electrosurgical generator.

## Description

The invention relates to an electrosurgical generator for operating an electrosurgical instrument. The invention also relates to an electrosurgical generator system and a method for operating an electrosurgical generator for operating an electrosurgical instrument.

Electrosurgical generators for operating electrosurgical instruments are generally known. Such generators are used as a power supply for the electrosurgical instruments connected to the generator. Such generators are also used for monitoring the correct operation of the connected electrosurgical instrument.

When using electrosurgical instruments that are powered by an electrosurgical generator, it is has to be assured that the instruments are sterile. For that reason solutions are known that disable the electrosurgical instruments, when the time of active use or a number of activations is exceeded.

Such a solution is known from US 6,237,604 B1. The document US 6,237,604 B1 describes an electrosurgical generator, which stores an instrument list (instrument catalog) of used instruments in a data storage of the electrosurgical generator, in which a time of active use and a number of uses for each instrument are recorded over time, which are connected to the generator. After connecting the electrosurgical instrument to the electrosurgical generator and activating the instrument, an instrument list of the generator is updated or a new entry is generated in the instrument list, when the instrument was not connected to the generator before. Thus, the usage data is not stored on the instrument, which simplifies the construction of the instrument. It is also known from US 6,237,604 B1 that the electrosurgical instruments will be disabled, if an allowed active usage time or an allowed number of activations of the instrument is exceeded.

Since the handheld electrosurgical instrument is sterile, the instrument should only be used for safety reasons for a short time, for instance one day, after opening or placing the instrument in an environment that is not completely sterile. However, a tracking of a usage time and number of activations has the disadvantage that the time, since when the instrument was used for the first time, does not necessary correspond to the active usage time of the instrument. Thus, a risk of contamination of the instrument is present, because the real passed time may be too long.

A second disadvantage of known solutions and in particular of the solution of US 6,237,604 B1 is that the devices for disabling the instruments are disabling the instruments, when the time of active use or number of uses is reached. Thus, it may happen that the active use time limit is reached, when the instrument is currently in use and the operator may be surprised by the shutdown.

Therefore, it is an object of the present invention to provide a solution that increases the safety of using an instrument connected to the generator. In particular, it is an object of the present invention to provide a solution, which reduces the risk of a contamination of the electrosurgical instrument connected to the electrosurgical generator and/or which improves the usability of the electrosurgical instrument connected to the electrosurgical generator.

According to a first aspect of the invention an electrosurgical generator for operating an electrosurgical instrument according to claim 1 is provided.

According to the first aspect, an electrosurgical generator for operating an electrosurgical instrument is provided. The electrosurgical generator is thus used for driving the electrosurgical instrument. The electrosurgical generator supplies the instrument with electrical power, for instance, from an electrical supply grid or from an electrical storage device, such as a battery.

Preferably, the generator is a high frequency generator that generates a high frequency supply current at the instrument terminal, in order to supply a connected instrument with a high frequency current and/or high frequency voltage.

The electrosurgical generator comprises an instrument terminal for connecting and electrically supplying the electrosurgical instrument. The instrument terminal can also be understood as a connector or port or connection socket. In operation the instrument is connected to the terminal in order to provide an electrical connection between the instrument and the generator. The generator is configured to supply the instrument with current or voltage electrically. The generator is connected for that purpose for example with an electrical supply grid and includes a power converter for power supply of the instrument. The power converter can also be referred to as switching device. Power converter are generally known. Power converters for converting alternating current to direct current are known as rectifiers. Power converters for converting direct current to alternating current are known as inverted rectifier or inverters. Power converters for converting a AC current into another AC current are known as frequency converters. Power converters for converting a direct current into another direct current are known as DC-to-DC converters. The generated alternating current or generated direct current can be higher, lower or inverted depending on the design of the converter or the requirements of the electrical load, respectively in the present case the electrosurgical instrument.

The electrosurgical generator further comprises an instrument detection device for detecting an electrosurgical instrument connected to the instrument terminal during operation of the electrosurgical generator. The instrument detection device is a device that is adapted to determine which individual instrument is connected at the instrument terminal. The instrument detection device is preferably a device that is adapted to identify a unique identifier, for example a bar code or the like, which is arranged on the electrosurgical instrument. It is understood that for this purpose the generator includes an identification device, such as a scanner, readout device, or the like. The instrument detection device may also preferably be a device that is adapted to receive a unique identifier by means of a data transmission from the electrosurgical instrument. It is also understood that for this purpose, the generator includes a data communication device or data exchange device, such as a data interface and the instrument includes a data transfer line for data exchange with the generator. The data transfer line may be a wired line. The data transfer line may also be a wireless transfers line, based on RFID, NFC or Bluetooth technology or the like.

The electrosurgical generator further comprises a control device for controlling a signaling device and a deactivation device, wherein the signaling device is configured to generate a warning indication.

The control device can also be referred to as controller or control unit. The control device is for example a processor of the electrosurgical generator, such as a CPU, a microcontroller or the like. The control device is signal connected with the signaling device and deactivation device, which means that the said devices are connected for data or signal transfer via data or signal lines. The control device can be understood as a controller that controls the signaling device and the deactivation device, which can be understood as actuators. The control device is adapted to generate control signals for controlling the signaling device and/or the deactivation device.

The signaling device is a device that is adapted to generate a warning indication, such as an optical or acoustical warning indication. The warning indication may be indicated at the generator. An example for an optical warning indication is a warning message shown on a display of the generator and an example for an acoustical warning indication is a sound signal that is generated by means of a sound generator, such as a speaker.

The deactivation device is a device that is configured to take an electrosurgical instrument connected to the instrument terminal out of operation or to keep the electrosurgical instrument out of operation. The deactivation device is for example a controllable switching unit that disables the power supply of the instrument. The deactivation device is in a second example a control unit that transmits a shut-off signal or a disabling signal to a receiver of the electrosurgical instrument. Both signals may disable the electrosurgical instrument for a further use. Keeping the electrosurgical instrument connected to the instrument terminal out of operation means, that if the instrument is already shut-off, the instrument cannot be activated anymore. Taking an electrosurgical instrument connected to the instrument terminal out of operation means that an actively used instrument is automatically shut off by the deactivation device.

The electrosurgical generator additionally comprises a data storage device coupled to the control device for storing a warning time and a switch-off time, the warning time and the switch-off time each being assigned to a detected instrument in order to assign an instrument-specific warning time and switch-off time to each detected instrument. The electrosurgical generator thus comprises a data store for storing the warning time and the switch-off. The data storage device is for example a non-volatile data storage. The warning time describes a time or time duration, when the warning indication shall be generated by means of the signaling device. The warning time can thus be understood as a first time threshold value or first time span. The warning time is for example a time duration of 10 hours. The switch-off time describes a time or time duration, when the instrument shall be deactivated by means of the deactivation device. The switch-off time can thus be understood as a second time threshold value or second time span. The shut-off time is for example a time duration of 12 hours. It is understood that in operation the switch-off time, for example 12 hours, is greater than the warning time, for example 10 hours. The warning time and the shut-off time are implemented in the data storage device as reference values for a comparison with an activation duration, that is determined by means of a time tracking device which is described below. Furthermore, it is suggested, that for each connected instrument connected to the generator an instrument-specific warning time and shut-off time will be assigned. This allows to assign different warning times and different shut-off times to different instruments. However, it is understood that also the same values for the warning time and the shut-off time may be implemented for each individual instrument. In a concrete implementation example, an instrument list or catalog may be stored in the data storage device, which includes the warning time and the shut-off time as entries for each individual instrument. The data storage for storing the warning time and/or the shut-off time may be the same data store that is used to store an instrument list or it is a different data storage than the storage of the instrument list.

The electrosurgical generator also comprises a time tracking device for determining an instrument-specific activation duration, wherein the activation duration is a duration of time detected from a first connection or a first activation of an electrosurgical instrument connected to the instrument terminal to an actual time value. The actual time value can also be understood as current time or present time at time of operating of the electrosurgical generator. The time tracking device can also be understood as a timer or a counter that records the passed time starting from the first activation or first connection of an instrument connected to the generator. The activation duration can thus be understood as a continuously determined time duration starting from the first activation or first connection of the instrument until an actual time. If for example an instrument was activated or connected to the generator two hours ago for the first time, the activation duration is two hours, regardless of whether the electrosurgical instrument was used or disconnected from the generator within this time. The activation duration thus describes an actual elapsed time value, measured from said first connection or first activation of the electrosurgical instrument until said actual time value.

The activation duration is detected for each detected instrument. Thus, it is suggested to track or record the passed time from said first activation or first connection for each instrument. Therefore, if different instruments are activated or connected to the generator at different times, the activation time of said instrument will be different.

The activation duration is recorded continuously and independently of an operating state of the electrosurgical instrument. It is thus suggested not to determine the active usage time of the instrument. It is in contrast suggested to record the passed time since a first activation or first connection of the instrument with the generator. The tracking or recording of the activation duration is for example not interrupted, when an instrument connected to the generator will be disconnected. The activation duration is thus a non-stop time measurement, which is independently tracked or recorded from the state of the connected instrument. Even if the instrument is disconnected from the generator or the instrument is not used, the activation duration will still be recorded or tracked by means of the time tracking device.

Preferably, the start of tracking the activation duration by means of the time tracking device is triggered by means of the instrument detection device. It is thus suggested that the instrument detection device determines or triggers the starting point of the activation duration for each connected instrument.

The control device of the electrosurgical generator triggers the warning indication by triggering the signaling device when the determined activation duration exceeds the instrument-specific warning time of the connected instrument. It is thus suggested that the control device generates a control signal for controlling the signaling device, when the instrument-specific warning time is reached or exceeded. In case the activation duration is equal or greater than the warning time, a warning indication is automatically generated by means of the signaling device.

The control device of the electrosurgical generator additionally or alternatively triggers the taking or keeping the connected instrument out of operation by controlling the deactivation device when the determined activation duration exceeds the instrument-specific switch-off time. It is thus suggestion that the control device generates a control signal for controlling the deactivation device, when the instrument-specific warning time is reached or exceeded. In case the activation duration is equal or greater than the shut-off time, the connected instrument is automatically shut-off by means of the deactivation device. In an example, an error indication can be generated that disables the instrument or disables the generator in order to take the connected instrument out of operation or in order to keeping the connected instrument out of operation.

Due to the continuously and independently of an operating state of the electrosurgical instrument recorded activation duration, it is ensured, that the electrosurgical instrument will not be used for a too long period of time. This way a solution is provided which increases the safety of using an instrument connected to the generator. In particular, the suggested solution reduces the risk of a contamination of the electrosurgical instrument connected to the electrosurgical generator.

Due to the implemented warning time, it is furthermore ensured that the operator of the electrosurgical instrument will not be surprised by an automaticalshut-off of the instrument, since a warning indication is generated, if the warning time is reached. This way a solution is provided which increases the safety of using an instrument connected to the generator. In particular, the suggested solution improves the usability of the electrosurgical instrument connected to the electrosurgical generator.

Thus, it is in general suggested to implement a single use activation counter in the electrosurgical generator, which continuously and independently tracks the activation duration of each instrument, independently of the operating state of the instrument.

Furthermore, it is generally understood that the activation duration can be reset, for example, when a used electrosurgical instrument was again sterilized.

Preferably, the signaling device includes an acoustic signaling device for outputting an acoustic signal as warning indication and/or the signaling device comprises an optical signaling device for outputting an optical signal or an optical message as warning indication. An example for an optical warning indication is a warning message shown on a display of the generator or an optical signal, which is generated by means of a colored lamp or LED at the generator, for instance a yellow or red LED is activated, when the warning time is reached. An example for an acoustical warning indication is a sound signal, which is generated by means of a sound generator, such as a loudspeaker.

Preferably, the data storage device is configured to store an instrument list in which instrument-specific information to the electrosurgical instruments connected to the instrument terminal are retrievably stored. The instrument list can also be understood as an instrument catalog. The instrument list preferably includes for each connected instrument at least one of the following entries or information: a unique instrument identifier, the warning time, the shut-off time, the first activation time, the first connection time, a time offset, the determined activation duration, a first flag indicating that the warning time is reached and a second flag indication that the shut-off time is reached. The instrument list or instrument catalog is retrievably stored in the data storage device, which means that the entries of the instrument list can be called and/or viewed by an authorized operator of the generator. The list can be called manually for example by means of an operating device, such as control buttons or a touch display, which are part of the electrosurgical generator. Furthermore, it is suggested that the entries of the instrument list are customizable and are therefore editable by the authorized operator. The unique instrument identifier is for example a unique code or ID assigned to an instrument, such as a unique hex code or the like. The first flag is for example a bit with the states true or false. The second flag is for example a bit with the states true or false.

Preferably, the instrument detection device is adapted to transmit a unique instrument identifier of the connected instrument to the data storage device for generating an instrument list. It is thus suggested, that the instrument detection device detects which instrument is connected to the instrument terminal or is activated. In order to create the document list the instrument detection device transmits the information of the detected instrument as an identifier to the data storage device. The identifier could be directly read out by the instrument detection device from the connected instrument or the identifier could be generated by the instrument detection device depending on a read out information at or from the connected instrument.

Preferably, the activation duration is an elapsed real time duration that is independent of a use duration or active operation duration of the connected instrument, wherein the use duration or active operation duration describes a time duration that indicates how long an instrument was actively used or was actively operated. It is thus suggested that the activation duration is a non-stop time measurement in real time, which is independent of the state of the connected instrument. Even if the instrument is disconnected from the generator or the instrument is not used, the activation duration will still be recorded.

Preferably, the electrosurgical generator further comprises a daytime device for detecting and/or displaying a current time and the time tracking device for determining the instrument-specific activation duration comprises a time basis that is independent of the daytime device in order to operate the time tracking device independent to the daytime device. The daytime device can be understood as clock that determines the present daytime, respectively the current time. The current time can be displayed at a display of the generator. Furthermore, it is suggested that the time tracking device has a time basis that is independent from detected current time. Due to the independent time basis of the time tracking device a manual adjustment of the current time at the generator has no impact on the tracked activation duration. Thus, an operator cannot manually adjust the current time at the generator in order to affect the activation time. This increases the safety.

Preferably, the time tracking device is configured to determine a time offset when the current time of the electrosurgical generator is manually adjusted and the time tracking device is configured to use the determined time offset for determining the instrument-specific activation duration. In case the current time is adjusted by the operator manually, the generator is thus adapted to track the manual adjustment of time by means of a time offset. If for example an operator sets the time on purpose two hours into the past, an offset of two hours will be stored in the generator, in order to take the adjustment of time into account when determining the activation duration.

Preferably, the first activation of the electrosurgical instrument connected to the instrument terminal is performable by means of an activation device coupled to the electrosurgical generator, wherein the activation device is preferably a pedal device and/or a hand switch. It is thus suggested that an activation device for activation the instrument is connected with the generator, wherein the activation device is preferably an external device, which is not part of the electrosurgical instrument. Preferably, the activation device is a pedal device, such as a foot pedal or the like. Preferably, the activation device is a hand switch, such as a button or toggle switch or the like. An external activation device, which is not part of the instrument, has the advantage that the complexity and the weight of the electrosurgical instrument can be reduced.

Preferably, the control device triggers the warning indication by triggering the signaling device when the determined activation duration exceeds the instrument-specific warning time of the connected instrument and when a connection of the instrument is detected and/or an activation of the instrument is detected. It is thus suggested to trigger the warning indication not directly when the warning time is reached or overstepped, but when one of the additional actions or events occur, namely a connection of the instrument is detection and/or an activation of the instrument is detected. It is understood that the generator includes means for detecting the connection of the instrument and/or for detecting the activation of the instrument. For example, a warning indication is directed generated, when the warning time is reached and when the used instrument is connected again to the generator or a warning indication is generated, when the warning time is reached and after the instrument is activated.

Preferably, the control device triggers the taking or keeping the connected instrument out of operation by controlling the deactivation device when the determined activation duration exceeds the instrument-specific switch-off time and when a connection of the instrument is detected and/or an activation of the instrument is detected. It is thus suggested to trigger the taking or keeping the connected instrument out of operation not directly when the shut-off time is reached or overstepped, but when one of the additional actions or events occur, namely a connection of the instrument is detection and/or an activation of the instrument is detected. It is understood that the generator includes means for detecting the connection of the instrument and/or for detecting the activation of the instrument. For example, an instrument is disabled or cannot be activated, when the shut-off time is reached and when the used instrument is connected again to the generator or an instrument is disabled or cannot be activated, when the shut-off time is reached and an activation of the instrument is detected.

According to a further aspect of the invention an electrosurgical generator system is provided, comprising an electrosurgical generator and an electrosurgical instrument connected to the electrosurgical generator, wherein the electrosurgical generator is embodied according to any one of the preceding or subsequent described embodiments. The electrosurgical generator system is thus a system that includes at least the electrosurgical generator as described hereinbefore or hereinafter and an electrosurgical instrument connected to the generator.

According to a second further aspect of the invention a method for operating an electrosurgical generator for operating an electrosurgical instrument is provided, wherein the electrosurgical generator comprises an instrument terminal for connecting and electrically supplying the electrosurgical instrument, an instrument detection device for detecting an electrosurgical instrument connected to the instrument terminal during operation of the electrosurgical generator, and a control device for controlling a signaling device and a deactivation device, wherein the signaling device is configured to generate a warning indication, and wherein the deactivation device is configured to take an electrosurgical instrument connected to the instrument terminal out of operation or to keep the electrosurgical instrument out of operation, and a data storage device coupled to the control device for storing a warning time and a switch-off time, the warning time and the switch-off time each being assigned to a detected instrument in order to assign an instrument-specific warning time and switch-off time to each detected instrument.

The method comprises the steps of:
Determining an instrument-specific activation duration with a time tracking device, wherein the activation duration is a duration of time detected from a first connection or a first activation of an electrosurgical instrument connected to the instrument terminal to an actual time value, and wherein an activation duration is detected for each detected instrument, wherein the activation duration is recorded continuously and independent of an operating state of the electrosurgical instrument.
Triggering the warning indication by controlling the signaling device with the control device when the determined activation duration exceeds the instrument-specific warning time of the connected instrument.
Triggering the taking or keeping the connected instrument out of operation by controlling the deactivation device with the control device when the determined activation duration exceeds the instrument-specific switch-off time.

The method thus comprises the main steps that for each electrosurgical instrument, which is connected to the generator the activation duration is tracked or recorded by means of the time tracking device. As described above the activation duration is continuously and independently recorded from the operating state of the electrosurgical instrument. The activation duration is thus independent from the operating state of the instrument. If the recorded activation duration reaches or exceeds the stored warning time, the control unit triggers a warning indication by controlling the signaling device. This way a warning is indicated at the generator before the instrument is shut down, when the activation duration reaches or exceeds the shut-off time. Thus, the automatical shut-off of the instrument is displayed beforehand with a warning. Thus, the safety of using an instrument connected to the generator is increased, since the operator is not surprised by the shut-off. Furthermore, due to the continuously and independently recorded activation duration, it is ensured that the instrument will not be used for a too long period of time and the contamination risk is reduced.

Preferably, the electrosurgical generator is embodied according to any one of the preceding or subsequent described embodiments, and/or the electrosurgical generator is part of the electrosurgical generator system according to the preceding or subsequent described embodiments.

As to the advantages, preferred embodiments and details of these further aspects and their preferred embodiments, reference is made to the corresponding advantages, preferred embodiments and details described above.

Preferred embodiments shall now be described with reference to the attached figures, in which
- Fig. 1: shows an electrosurgical generator system with an electrosurgical generator and an electrosurgical instrument connected to the electrosurgical generator according to an embodiment.
- Fig. 2: shows a schematic construction of an electrosurgical generator according to the invention.
- Fig. 3: shows the time tracking principle according to state of the art.
- Fig. 4: shows the time tracking principle according to the invention.
- Fig. 5: shows a schematic flow chart of the method for operating an electrosurgical generator for operating an electrosurgical instrument.

In the figures, elements with the same or comparable functions are indicated with the same reference numerals.

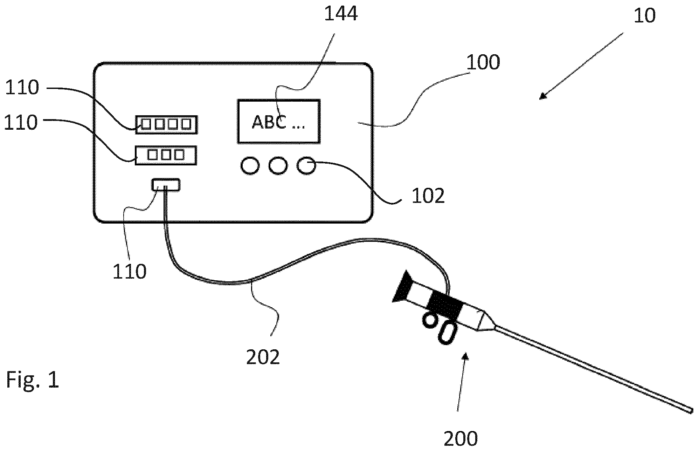

Figure 1 shows an electrosurgical generator system 10 with an electrosurgical generator 100 and an electrosurgical instrument 200 connected to the electrosurgical generator 100. The electrosurgical instrument 200 is connected with the electrosurgical generator 100 at the instrument terminal 110 by mean of a connection line 202. The connection line 202 may include supply lines and a data transfer line, which are not shown in Fig. 1. The supply line are used to supply the instrument 200 with electrical power and the data transfer line is used to exchange data between the instrument and the generator. The generator may also include several instrument terminals, for example a neutral instrument terminal, a unipolar instrument terminal and a bipolar instrument terminal. The electrosurgical instrument 200 includes a shaft having an active electrode at the end of the shaft. The shaft is attached to a handle of the electrosurgical instrument. The generator 100 includes a display 144 for displaying text and/or symbols and/or graphics, which is adapted to display an optical warning indication. The generator 100 also includes an operating device 102 which is illustrated as three buttons. The operating device 102 may also be a touch display or the like. The electrosurgical generator 100 is used for operating the electrosurgical instrument 200.

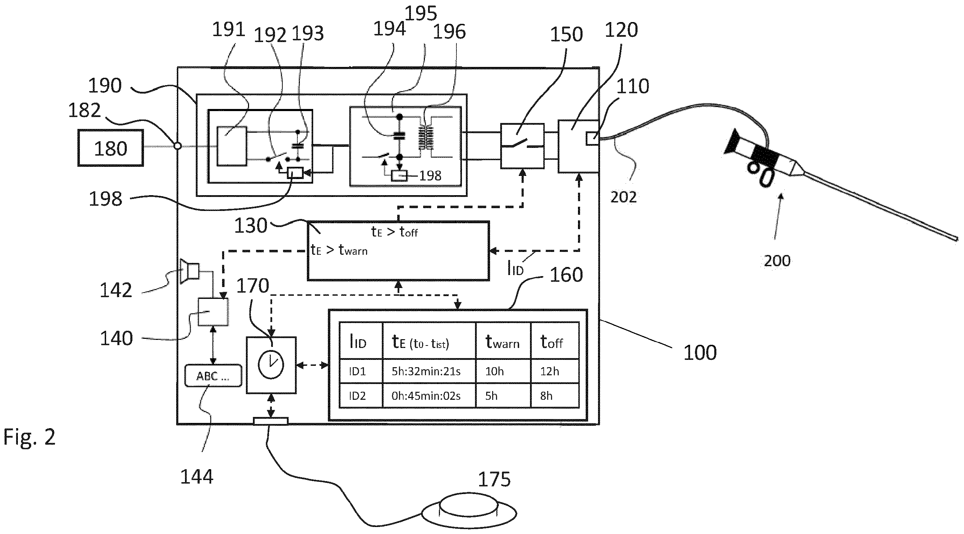

Figure 2 shows a schematic construction of an electrosurgical generator 100 in more detail.

The generator 100 includes an instrument terminal 110 for connecting and electrically supplying the electrosurgical instrument 200. For that purpose, the generator 100 includes a supply terminal 182 for connecting the generator 100 with an electrical supply grid 180. The generator 100 includes a power converter 190, which is used to convert the input current and the input voltage of the supply grid into a suitable operation current and/or operation voltage at the instrument terminal 110. The power converter 190 can also be understood as a switching power supply. Power converters for converting alternating current to direct current are known as rectifiers. Power converters for converting direct current to alternating current are known as inverted rectifier or inverters. Power converters for converting one AC current to another AC current are known as frequency converters. Power converters for converting one direct current to another direct current are known as DC-to-DC converters. The generated alternating current or generated direct current can be higher, lower or inverted depending on the design of the converter or the requirements of the electrical load, respectively the electrosurgical instrument. The shown power converter is an inverter that is adapted to convert the AC current from the grid into an AC operation current at the terminal 110 for electrical supplying the instrument 200 with an alternating current. However it is understood, that in case the instrument works with direct current, the power converter would be implemented as a DC-to-DC converter. The power converter 190 includes a rectifier device 191 in order to rectify the drawn alternating voltage from the grid into an intermediate circuit DC voltage. The capacitor 193 is an intermediate capacitor that is used as temporal energy buffer. The power converter 190 also includes a converter device 195 for generating the operation current and/or operation voltage at the instrument terminal 110. The converter device 195 for example includes a semiconductor switches such as IGBTs, MOSFETS or the like, which are arranged in a half-bridge topology or the like. The converter 195 may also include a transformer 196, for example a step-up-transformer, in order to step up the voltage at the instrument terminal into a voltage with a desired amplitude and or frequency. In general, the power converter 190 is adapted to generate a high frequency current for supplying the instrument 200 with power. The power converter 190 also includes driver circuits 197, 198 for controlling electrical switches, for example the electrical switch 192. The driver circuits 197, 198 and the switches illustrate that the power converter 190 may include a step-up-converter, a step-down-converter or the like, depending on the type of power converter 190. In case the power converter 190 is an AC-to-AC converter the driver circuits 197, 198 and the switches 192 illustrate that the power converter 190 includes controllable switches in order to generate the output current and output current at the instrument terminal 110. The rectifier 190 is for example a passive or active rectifier.

The generator 100 also includes an instrument detection device 120 for detecting an electrosurgical instrument 200 connected to the instrument terminal 110 during operation of the electrosurgical generator. The detection device 120 for example includes a data interface and is adapted to receive information from the connected instrument that is connected to the instrument terminal 110.

The generator 100 includes a control device 130 for controlling a signaling device 140 and a deactivation device 150.

The signaling device 140 is configured to generate a warning indication. The signaling device 140 thus may include an acoustic signaling device 142, namely a speaker, and/or an optical signaling device, namely a display 142.

The deactivation device 150 is configured to take an electrosurgical instrument 200 connected to the instrument terminal 110 out of operation or to keep the electrosurgical instrument 200 out of operation. In the showed embodiment the deactivation device 150 includes a controllable switch that interrupts the power supply of the instrument 200 connected to the terminal 110 of the generator. The deactivation device 150 may also be a data interface that transmits a shut-off signal to the instrument over a data transfer line.

The electrosurgical generator 100 also comprises a data storage device 160 coupled to the control device 130 for storing a warning time t_{warn} and a switch-off time t_{off}, the warning time and the switch-off time each being assigned to a detected instrument in order to assign an instrument-specific warning time and switch-off time to each detected instrument.

The electrosurgical generator 100 also comprises a time tracking device 170 for determining an instrument-specific activation duration t_{E}, wherein the activation duration t_{E} is a duration of time detected from a first connection t₀ or a first activation to of an electrosurgical instrument 200 connected to the instrument terminal 110 to an actual time value tᵢₛₜ, and wherein an activation duration t_{E} is detected for each detected instrument.

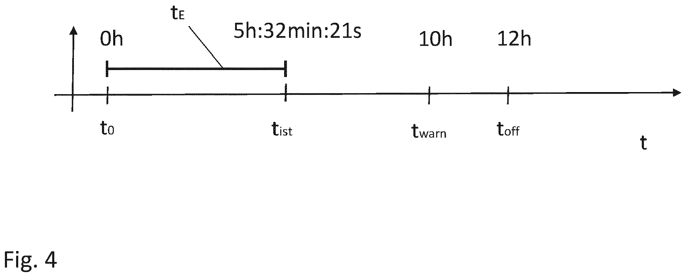

The activation duration t_{E} is recorded continuously and independently of an operating state of the electrosurgical instrument 200, which is showed in Figure 4 as an example. The activation duration t_{E} is an elapsed real time duration that is independent of a use duration or active operation duration of the connected instrument, wherein the use duration or active operation duration describes a time duration that indicates how long an instrument was used or was actively operated.

The control device 130 triggers the warning indication by triggering the signaling device 140 when the determined activation duration t_{E} exceeds the instrument-specific warning time of the connected instrument t_{E} > t_{warn}.

The control device 130 also triggers the taking or keeping the connected instrument out of operation by controlling the deactivation device 150 when the determined activation duration exceeds the instrument-specific switch-off time t_{E} > t_{off}.

Furthermore, figure 2 shows that the signaling device 140 includes an acoustic signaling device 142 for outputting an acoustic signal as warning indication, namely a loudspeaker and that the signaling device 140 comprises an optical signaling device 144 for outputting an optical signal or an optical message as warning indication, namely a display.

The data storage device 160 is configured to store an instrument list in which electrosurgical instruments connected to the instrument terminal 110 are retrievably stored. The instrument list includes unique identifiers ID1, ID2, the determined activation duration t_{E} wherein the activation duration t_{E} is the duration of time detected from a first connection to or a first activation to of an electrosurgical instrument 200 connected to the instrument terminal 110 to an actual time value tᵢₛₜ. The instrument list also includes the instrument specific warning times 10h and 5h and the instrument specific shut-off times 12h and 8h, wherein h indicates hours.

The instrument detection device 120 is adapted to transmit a unique instrument identifier I_{ID} of the connected instrument 110 to the data storage device 160 and/or to the control device 130 for generating the instrument list.

The electrosurgical generator 100 further comprises a daytime device that is not shown in figure 2 for detecting and/or displaying a current time. The time tracking device 170 for determining the instrument-specific activation duration t_{E} comprises a time basis that is independent of the daytime device in order to operate the time tracking device 170 independent to the daytime device.

The first activation to of the electrosurgical instrument 200 connected to the instrument terminal 110 is performable by means of an activation device 175 coupled to the electrosurgical generator 100, wherein the activation device is an external device which is not part of the electrosurgical instrument, namely a pedal device for foot activation.

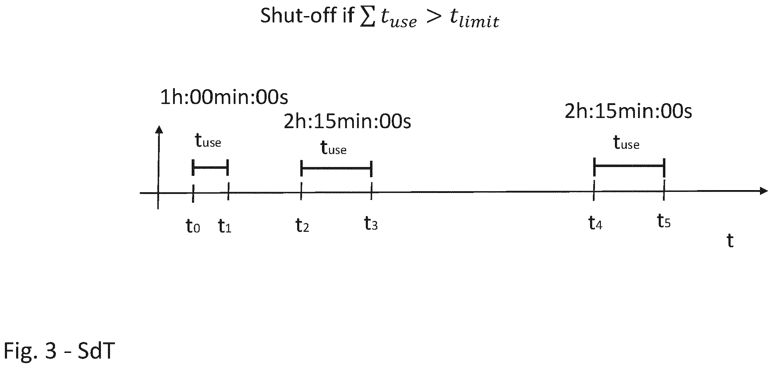

Figure 3 shows the time tracking principle according to the state of the art. In the state of the art the use duration or active operation duration tᵤₛₑ of the connected instrument is tracked. The use duration or active operation duration describes a time duration that indicates how long an instrument was actively used or was actively operated. If for example an instrument is actively used for 1 hour from the time point to to t₁ the tracking of the use duration tᵤₛₑ will be stopped although the real time t will further increase. Later on at time point t₂ the instrument is actively used again until t₃ for 2h and 15min. Again later, at time point t₄ the instrument actively used until t₅ is reached for 2h and 15min. The total use duration tᵤₛₑ would thus be the sum of the overall usage time, thus in the example 5h:30min:00s. The actual time t is thus much larger than the total use duration tᵤₛₑ, since the usage time is not tracked in the pauses between the usages.

Figure 4 shows in contrast to figure 3 the time tracking principle according to the invention. In contrast to figure 3 the figure 4 shows that the activation duration t_{E} is recorded continuously and independently of an operating state of the electrosurgical instrument 200. The time detection starts at the time t₀, when the instrument 200 is connected to the generator 100 for the first time or is activated for the first time. The activation duration t_{E} is recorded continuously and independently from the operating state of the electrosurgical instrument 200. The activation duration t_{E} is thus an elapsed real time duration that is independent of a use duration tᵤₛₑ or active operation duration tᵤₛₑ as showed figure 3.

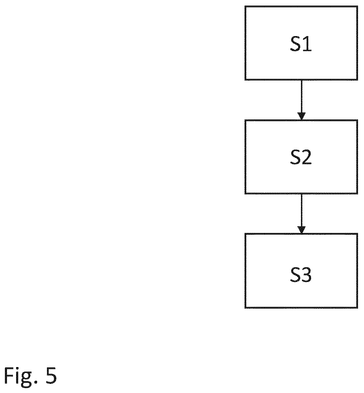

Figure 5 shows a schematic flow chart of the method for operating an electrosurgical generator for operating an electrosurgical instrument.

In step S1 determining an instrument-specific activation duration t_{E} with a time tracking device 170 is performed, wherein the activation duration t_{E} is a duration of time detected from a first connection to or a first activation to of an electrosurgical instrument 200 connected to the instrument terminal 110 to an actual time value tᵢₛₜ, and wherein an activation duration t_{E} is detected for each detected instrument, wherein the activation duration t_{E} is recorded continuously and independently of an operating state of the electrosurgical instrument 200.

In step S2 triggering a warning indication by controlling a signaling device 140 with a control device 130 is performed when the determined activation duration t_{E} exceeds an instrument-specific warning time t_{warn} of the connected instrument t_{E} > t_{warn} .

In step S3 triggering S3 a taking or keeping a connected instrument out of operation is performed by controlling a deactivation device 150 with the control device 130 when the determined activation duration exceeds the instrument-specific switch-off time t_{E} > t_{off}.

The generator, generator system and method described above offer a number of other advantages, which are summarized below:
- The complexity and the weight of the handheld electrosurgical instruments can be reduced, since all components for monitoring and disabling the electrosurgical instrument are provided in the generator but not in the electrosurgical instrument.
- A safer operation of the handheld electrosurgical instruments is provided, because the shut-off of the electrosurgical instrument is indicated with a warning. The user of the electrosurgical instrument will thus not be surprised by an unexpected shut-off.
- The risk of unsterile use due to a too long use of the device is reduced.

### Reference sign list

- 10: Electrosurgical generator system
- 100: Electrosurgical generator
- 102: Operating device
- 110: Instrument terminal
- 120: Instrument detection device
- 130: Control device
- 140: Signaling device
- 142: Acoustic signaling device
- 144: Optical signaling device
- 150: Deactivation device
- 160: Data storage device
- 170: Time tracking device
- 175: Activation device
- 180: Electrical supply grid
- 182: Supply terminal
- 190: Power converter
- 191: Rectifier device
- 192: Switch
- 193, 194: Capacitor
- 195: Converter device
- 196: Transformer
- 197, 198: Driver circuit
- 200: Electrosurgical instrument
- 202: Connection line

## Claims

1. An electrosurgical generator (100) for operating an electrosurgical instrument (200), comprising:
- an instrument terminal (110) for connecting and electrically supplying the electrosurgical instrument (200),
- an instrument detection device (120) for detecting an electrosurgical instrument (200) connected to the instrument terminal (110) during operation of the electrosurgical generator,
- a control device (130) for controlling a signaling device (140) and a deactivation device (150), wherein the signaling device (140) is configured to generate a warning indication, and wherein the deactivation device (150) is configured to take an electrosurgical instrument connected to the instrument terminal (110) out of operation or to keep the electrosurgical instrument out of operation, **characterized in that** the electrosurgical generator (100) additionally comprises
- a data storage device (160) coupled to the control device (130) for storing a warning time (t_{warn}) and a switch-off time (t_{off}), the warning time and the switch-off time each being assigned to a detected instrument in order to assign an instrument-specific warning time and switch-off time to each detected instrument,
- a time tracking device (170) for determining an instrument-specific activation duration (t_{E}), wherein the activation duration (t_{E}) is a duration of time detected from a first connection (to) or a first activation (to) of an electrosurgical instrument (200) connected to the instrument terminal (110) to an actual time value (tᵢₛₜ), and wherein an activation duration (t_{E}) is detected for each detected instrument, and
- the activation duration (t_{E}) is recorded continuously and independently of an operating state of the electrosurgical instrument (200), and
- the control device (130) triggers the warning indication by triggering the signaling device (140) when the determined activation duration (t_{E}) exceeds the instrument-specific warning time of the connected instrument (t_{E} > t_{warn}), and/or
- the control device (130) triggers the taking or keeping the connected instrument out of operation by controlling the deactivation device (150) when the determined activation duration exceeds the instrument-specific switch-off time (t_{E} > t_{off}).

2. Electrosurgical generator (100) according to claim 1, wherein
- the signaling device (140) includes an acoustic signaling device (142) for outputting an acoustic signal as warning indication and/or
- the signaling device (140) comprises an optical signaling device (144) for outputting an optical signal or an optical message as warning indication.

3. Electrosurgical generator (100) of claim 1 or 2, wherein the data storage device (160) is configured to store an instrument list in which instrument-specific information to the electrosurgical instruments connected to the instrument terminal (110) are retrievably stored, preferably the instrument list includes for each connected instrument at least one of the following entries or information: a unique instrument identifier, the warning time, the shut-off time, the first activation time, the first connection time, a time offset, the determined activation duration, a first flag indicating that the warning time is reached and a second flag indication that the shut-off time is reached.

4. Electrosurgical generator (100) according to any one of the preceding claims, wherein the instrument detection device (120) is adapted to transmit a unique instrument identifier (I_{ID}) of the connected instrument (110) to the data storage device (160) for generating an instrument list.

5. Electrosurgical generator (100) according to any one of the preceding claims, wherein the activation duration (t_{E}) is an elapsed real time duration that is independent of a use duration or active operation duration of the connected instrument, wherein the use duration or active operation duration describes a time duration that indicates how long an instrument was used or was actively operated.

6. Electrosurgical generator (100) according to any one of the preceding claims, wherein the electrosurgical generator (100) further comprises a daytime device for detecting and/or displaying a current time, and the time tracking device (170) for determining the instrument-specific activation duration (t_{E}) comprises a time basis that is independent of the daytime device in order to operate the time tracking device (170) independent to the daytime device.

7. Electrosurgical generator (100) according to claim 6, wherein the time tracking device (170) is configured to determine a time offset when the current time of the electrosurgical generator is manually adjusted and the time tracking device (170) is configured to use the determined time offset for determining the instrument-specific activation duration (t_{E}).

8. Electrosurgical generator (100) according to any one of the preceding claims, wherein the first activation (to) of the electrosurgical instrument (200) connected to the instrument terminal (110) is performable by means of an activation device (175) coupled to the electrosurgical generator (100), wherein the activation device is preferably an external device which is not part of the electrosurgical instrument, and wherein the activation device (175) is preferably a pedal device and/or a hand switch.

9. Electrosurgical generator (100) according to any one of the preceding claims, wherein
- the control device (130) triggers the warning indication by triggering the signaling device (140) when the determined activation duration (t_{E}) exceeds the instrument-specific warning time of the connected instrument (t_{E} > t_{warn}) and when a connection of the instrument is detected and/or an activation of the instrument is detected; and/or
- the control device (130) triggers the taking or keeping the connected instrument out of operation by controlling the deactivation device (150) when the determined activation duration exceeds the instrument-specific switch-off time (t_{E} > t_{off}) and when a connection of the instrument is detected and/or an activation of the instrument is detected.

10. Electrosurgical generator system (10), comprising an electrosurgical generator (100) and an electrosurgical instrument (200) connected to the electrosurgical generator (100), wherein the electrosurgical generator (100) is embodied according to any one of the preceding claims 1 to 9.

11. A method for operating an electrosurgical generator (100) for operating an electrosurgical instrument (200), the electrosurgical generator (100) comprising:
- an instrument terminal (110) for connecting and electrically supplying the electrosurgical instrument (200),
- an instrument detection device (120) for detecting an electrosurgical instrument (200) connected to the instrument terminal (110) during operation of the electrosurgical generator,
- a control device (130) for controlling a signaling device (140) and a deactivation device (150), wherein the signaling device (140) is configured to generate a warning indication, and wherein the deactivation device (150) is configured to take an electrosurgical instrument connected to the instrument terminal (110) out of operation or to keep the electrosurgical instrument out of operation, and
- a data storage device (160) coupled to the control device (130) for storing a warning time (t_{warn}) and a switch-off time (t_{off}), the warning time and the switch-off time each being assigned to a detected instrument in order to assign an instrument-specific warning time and switch-off time to each detected instrument, **comprising the steps of:**
- determining (S1) an instrument-specific activation duration (t_{E}) with a time tracking device (170), wherein the activation duration (t_{E}) is a duration of time detected from a first connection (to) or a first activation (to) of an electrosurgical instrument (200) connected to the instrument terminal (110) to an actual time value (tᵢₛₜ), and wherein an activation duration (t_{E}) is detected for each detected instrument, wherein the activation duration (t_{E}) is recorded continuously and independently of an operating state of the electrosurgical instrument (200), and
- triggering (S2) the warning indication by controlling the signaling device (140) with the control device (130) when the determined activation duration (t_{E}) exceeds the instrument-specific warning time of the connected instrument (t_{E} > t_{warn}), and/or
- triggering (S3) the taking or keeping the connected instrument out of operation by controlling the deactivation device (150) with the control device (130) when the determined activation duration exceeds the instrument-specific switch-off time (t_{E} > t_{off}).

12. Method according to claim 11, wherein the electrosurgical generator (100) is embodied according to any one of the preceding claims 1 to 9, and/or the electrosurgical generator (100) is part of the electrosurgical generator system (10) according to claim 10.
